Europäisches Patentamt

European Patent Office

Office européen des brevets

(11) Publication number: **0 252 618 B1**

(12) **EUROPEAN PATENT SPECIFICATION**

(45) Date of publication of patent specification: **25.03.92** (51) Int. Cl.⁵: **A61M 16/08**, A62B 7/00, A61M 16/00

(21) Application number: **87305210.4**

(22) Date of filing: **12.06.87**

(54) **Resuscitator.**

(30) Priority: **07.07.86 US 882773**

(43) Date of publication of application:
**13.01.88 Bulletin 88/02**

(45) Publication of the grant of the patent:
**25.03.92 Bulletin 92/13**

(84) Designated Contracting States:
**AT BE CH DE ES FR GB GR IT LI LU NL SE**

(56) References cited:
**DE-B- 1 491 631**
**GB-A- 2 063 687**
**GB-A- 2 145 335**
**US-A- 4 106 502**

(73) Proprietor: **Bauman, Jack**
**1677 San Onofre Drive**
**Pacific Palisades California 90272(US)**

(72) Inventor: **Bauman, Jack**
**1677 San Onofre Drive**
**Pacific Palisades California 90272(US)**

(74) Representative: **Hughes, Brian Patrick et al**
**Graham Watt & Co. Riverhead**
**Sevenoaks, Kent TN13 2BN(GB)**

## Description

BACKGROUND OF THE INVENTION

This invention relates generally to resuscitation of patients, as during heart attacks, shock, fainting, etc.; more particularly it concerns improved apparatus, characterized by high reliability, simplicity of construction, ease of use and safety against infection, and incorporation of multiple safety features.

In the past, mouth-to-mouth resuscitation was believed to be necessary to provide required inhalation and exhalation of patients undergoing shock, heart attacks, etc.; however, the risk and danger of infection to the administrator of resuscitation is now recognized as serious, indeed critical, and to be avoided at all times. There is need for a simple, safe and inexpensive resuscitation apparatus that is capable of easy manual use.

GB 2145335 discloses resuscitation apparatus in which a squeezable air bag or receptacle supplies air to a face mask, the apparatus including three flap valves which operate to control the flow of air to the air bag, from the air bag to the face mask and from the face mask to the atmosphere.

SUMMARY OF THE INVENTION

It is a major object of the invention to provide improved apparatus meeting the above need. Basically, the resuscitation apparatus of the invention comprises a manually collapsible air receptacle having an air discharge outlet, first and second tubing sections connected in series and having air inlet means connectible in air passing relation with said receptacle discharge outlet, and an air outlet connectible in air passing relation with a mask to be placed against the patient's face, and first and second flap valves positioned in at least one tubing section so that the first flap valve opens and passes air to said receptacle when the second flap valve is closed, and the second flap valve opens and passes air from the receptacle to the mask when the receptacle is squeezed and the first flap valve is closed, characterised by air pressure responsive indicator means carried by said first section to indicate the extent of pressure build-up between the second valve and said tubing section air outlet, the indicator means including a pressure sensitive part movably carried by said first section, and means indicating the displacement of said part in response to air pressure build-up.

As will be seen, the air receptacle typically comprises a bellows that is substantially completely hand-collapsible, so that a minimum size receptacle may be provided. Also, the tubing sections may be simplified in this construction and assembly, by use of first, second and third sections which are T-shaped, and each having a side opening and end openings, the side opening of the second section registered with one end opening of the third section. This also simplifies the flap valve construction in that the first flap valve controls the side opening of the second section, and the second flap valve controls an end opening of the first section that is in registration with the first section.

Another, preferred, object is the provision of a re-entrant duct extending into one tubing section and defining an air discharge port that is open to communicate with said air outlet when said second flap valve is closed, the second flap valve including a flap, and said air discharge port being closed by said flap of the second flap valve when said second flap valve is open. A cap may be provided to control escape of air from the re-entrant air discharge duct extending into one tubing section and defining an air discharge port communicating with said air outlet, and a cap controlling escape of air from the re-entrant duct and movable to increase or decrease the effective size of said air discharge port.

Finally, an air pressure relief valve may be provided to prevent excess pressure build-up, as will be seen.

These and other objects and advantages of the invention, as well as the details of an illustrative embodiment, will be more fully understood from the following specification and drawings, in which:

DRAWING DESCRIPTION

Fig. 1 is a section showing interconnected tubing sections and associated flap valves;
Fig. 2 is a perspective view of a collapsible air receptacle connectible to the Fig. 1 tubing sections;
Fig. 3 is an end view on lines 3-3 of Fig. 1;
Fig. 4 is an enlarged frontal view of a valve flapper;
Fig. 5 is a section taken on lines 5-5 of Fig. 4;
Fig. 6 is a section showing a modified tubing section;
Fig. 7 is a top plan view taken on lines 7-7 of Fig. 6;
Fig. 8 is a view showing an alternative collapsible receptacle; and
Fig. 9 shows that receptacle in collapsed condition.

DETAILED DESCRIPTION

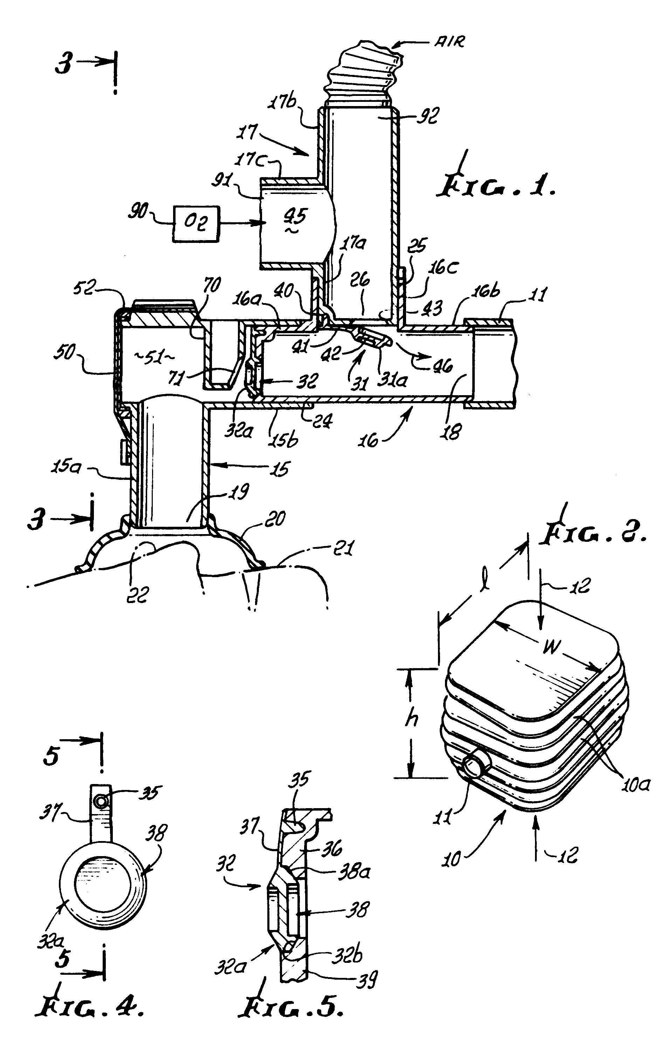

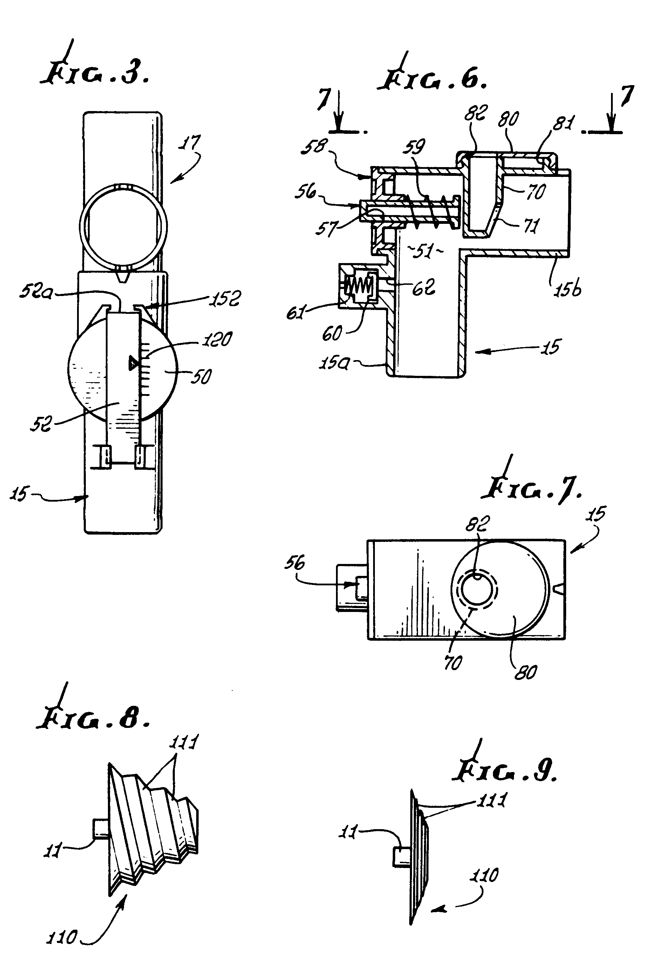

In Figs. 1 and 2, a manually collapsible air receptacle 10 has an air discharge outlet 11 attached to a tubing assembly. The receptacle has a bellows-like configuration to be easily hand-held, and collapsed endwise, in a direction indicated by arrow 12. Note accordion-like pleats 10a. The receptacle may have height "h", length "l", width "w" dimensions for convenient hand manipulation about as follows:

h = height 4 inches (10.16 cm)
l = about 4 inches (10.16 cm)
w = about 4 inches (10.16 cm)

Almost complete collapse of the receptacle can thereby be achieved.

The tubing assembly includes first, second and third tubular sections 15, 16 and 17, joined together in series, as shown. They define air inlet means, as at 18, connectible in air passing relation with the receptacle outlet 11, and an air outlet 19 connectible in air passing relation with a mask 20 configured to be placed against the patient's face 21, for supplying air to the patient as via his nose 22, for resuscitation. As shown, tubular section 15 is elbow shaped, and has tubular legs 15a and 15b; and sections 16 and 17 are alike and have tee shape. Their likeness enables reduction in molding costs, all sections consisting of molded plastic, for example. Section 16 has tubular legs 16a and 16b, and tubular stem 16c, leg 16a having telescopic interfit at 24 with leg 15b. Section 17 has tubular legs 17a and 17b, and tubular stem 17c. Leg 17a has telescopic interfit at 25 with stem 16c. Thus, the side opening of second section 16 defined by stem 16c registers with the end opening 26 defined by leg 17a of third section 17. The telescopic interfits at 24 and 26 may be suitably bonded together to provide rigid connections.

Also provided are first and second flap valves generally indicated at 31 and 32, and positioned in at least one tubing section, so that the first flap valve 31 opens and passes intake air to the receptacle to inflate same when the second flap valve is closed; and so that the second flap valve 32 opens and passes air from the receptacle (upon squeezing thereof) to the mask 20 via outlet 19, and while flap valve 31 is closed. In this regard, the flap valves typically include normally closed, like flaps 31a and 32a, flap 32a illustrated in the closed position in Fig. 1, and flap 31a illustrated in open position in that view. Figs. 4 and 5 show flap 32a as having an anchor 35 penetrating and cemented to wall portion 36 of tubular section 16; a thin cantilever flat spring arm 37, and a disc 38 carried by the arm and annularly tapered at 38a to fit its annular seat 32b in wall 39, when the valve is closed. Valve flapper 31a is similarly configured, with parts 40, 41, 42 and 43 corresponding to parts 35, 37, 38 and 39. The flapper may consist of DELRIN plastic material, for example.

When the receptacle 10 is allowed to expand (after its forcible compression) inflating air enters side inlet 45 in section 17, opens flap valve 31, and flows to the receptacle as indicated by arrow 46. At this time, flap valve 32 is closed. When the receptacle is squeezed, flap valve 31 closes, and valve 32 opens to pass air to outlet 19 and to the mask 20 to resuscitate the patient.

Air pressure indicator means may be carried by the tubular section assembly, to indicate the extent of pressure build-up between valve 32 and outlet 19. In the example shown in Fig. 1, the indicator comprises a movable pressure sensitive part in the form of a balloon membrane 50 exposed to the interior 51 of the elbow, an an indicator such as a slider 52 attached to the membrane. Increased pressure at 51 expands the membrane 50 to the left in Fig. 1, pushing the slider 52 to the left. A visible indication of pressure increase is thereby achieved, and indicia on the slider retainer 53 may indicate the degree of pressure increase, on a marker as the slider moves past the indicia. In Figs. 6 and 7, the indicator takes the form of a plunger 56 slidable in a bore 57 formed by an insert 58 attached to the elbow. The extent of plunger travel to the left, resisted by a spring 59, indicates the extent of pressure build-up at 51.

A safety valve may be provided to allow escape of excess pressure so that urging to the lungs of a patient may be prevented. As shown, a check valve part 60 is spring urged at 61 to close outlet 62. Part 60 is pushed open by excess air pressure to allow air escape.

Also shown in Fig. 1 is a re-entrant duct 70 extending into leg 15b of section 15. It defines an air discharge port 71 that is open to communicate with the air outlet 19 upon exhalation by the patient, flap valve 32 then being closed against seat 32b. Exhaled lung air then escapes via duct 70. Alternately, port 71 is closed by flap 32a when the second flap valve 32 is open, to allow air to flow from the squeezed receptacle to the mask 20. Flap 32a thus performs two functions. Note that air may flow about the re-entrant duct in each position of the flap 32a, for minimum flow restriction.

In Figs. 6 and 7, an enlarged cap 80 controls the effective size of discharge port 71, by controlling the escape of air from the re-entrant duct 70. To this end, the cap 80 fits annularly over an annular neck 81 on the leg 16b; and the cap has an opening 82 thereon that variably registers with the duct 70 as the cap is rotated on the enlarged neck. This serves as a control for air escape upon natural exhalation by the patient, so that positive end expiratory pressure (PEEP) may be achieved if desired.

In Fig. 1, a source of oxygen 90 may be connected with the inlet 45, so that supplied $O_2$ enters via inlet 91 for flow to the receptacle and reservoir tubing 92, and later to the patient. An oxygen flow rate of as little as 8 liters per minute will supply 100% oxygen to the patient.

Fig. 8 shows an alternative conically shaped receptacle 110, for air or oxygen, for use in place of receptacle 10. Its pleats 111 allow its complete endwise collapse, manually, as shown in Fig. 9. Thus maximal air discharge is achieved; and also high compactness is achieved for storage, as in a user's pocket. See part 11.

## Claims

1. A resuscitator comprising a manually collapsible air receptacle (10) having an air discharge outlet (11), first and second tubing sections (15, 16) connected in series and having air inlet means (18) connectible in air passing relation with said receptacle discharge outlet (11), and an air outlet (19) connectible in air passing relation with a mask (20) to be placed against the patient's face, and first and second flap valves (31, 32) positioned in at least one tubing section (16) so that the first flap valve (31) opens and passes air to said receptacle (10) when the second flap valve (32) is closed, and the second flap valve (32) opens and passes air from the receptacle (10) to the mask (20) when the receptacle is squeezed and the first flap valve (31) is closed, characterised by air pressure responsive indicator means carried by said first section (15) to indicate the extent of pressure build-up between the second valve (32) and said tubing section air outlet (19), the indicator means including a pressure sensitive part (50 or 56) movably carried by said first section, and means indicating the displacement of said part in response to air pressure build-up.

2. A resuscitator as claimed in claim 1, wherein said air receptacle comprises a bellows (10, 110) that is substantially completely collapsible.

3. A resuscitator as claimed in claim 1, wherein said one tubing section is an elbow.

4. A resuscitator as claimed in claim 1, wherein said part comprises a balloon (50) or a plunger (56).

5. A resuscitator as claimed in any preceding claim, including said mask (20) attached to said first section at said air outlet end thereof.

6. A resuscitator as claimed in any preceding claim, including a re-entrant duct (70) extending into one tubing section (15) and defining an air discharge port (71) that is to communicate with said air outlet (19) when said second flap valve (32) is closed, the second flap valve including a flap (32a), and said air discharge port (71) being closed by said flap (32a) of the second flap valve when said second flap valve (32) is open.

7. A resuscitator as claimed in claim 1, including a third tubing section (17), the second (16) and third (17) sections being T-shaped and each having a side opening and end openings, the side opening of the second section (16) registered with one end opening of the third section (17).

8. A resuscitator as claimed in claim 7, wherein the first flap valve (31) controls the side opening of the second section (16), and the second flap valve (32) controls an end opening of the second section that is in registration with the first section.

9. A resuscitator as claimed in claim 7, including a source of oxygen (90) in communication with the third section (17) via its side opening (91) or its other end opening (92).

10. A resuscitator as claimed in claim 1, including a re-entrant air discharge duct (70) extending into one tubing section (15) and defining an air discharge port (71) communicating with said air outlet (19), and a cap (80) controlling escape of air from the re-entrant duct and movable to increase or decrease the effective size of said air discharge port.

11. A resuscitator as claimed in claim 2, wherein the bellows (110) has conical shape, enabling substantially complete, endwise, manually effected collapse, in use, and also for storage.

## Revendications

1. Appareil de réanimation comprenant une chambre à air (10) écrasable manuellement, présentant un orifice de décharge d'air (11), des première et seconde sections de tube (15,16) reliées en série et présentant un orifice d'entrée d'air (18) pouvant être relié à l'orifice de sortie de décharge d'air (11) de la chambre à air, en laissant passer l'air à travers lui, et un orifice de sortie d'air (19) pouvant être relié, pour laisser passer l'air, à un masque (20) destiné à être placé sur la face d'un patient, et des premier et second clapets (31,32) disposés dans au moins une section de tube (16) de telle manière que le premier clapet (31) s'ouvre et laisse passer l'air en direction de la chambre (10), tandis que le second clapet (32) est fermé, et que le second clapet (32) s'ouvre et laisse passer l'air à partir de la chambre (10) et en direction du masque (20), lorsque la chambre est comprimée et que le premier clapet (31) est fermé, caractérisé en ce qu'il comprend un moyen indicateur sensible à la pression d'air, porté par la première section (15), afin d'indiquer la valeur de la pression créée entre le second clapet (32) et l'orifice de sortie d'air (19) de cette première section de tube, ce moyen indicateur comportant une partie sensible à la pression (50 ou 56) porté d'une manière mobile par la première section de tube, et un moyen indiquant le déplacement de cette partie en réponse à la création de la pression d'air.

2. Appareil suivant la revendication 1 caractérisé en ce que la chambre à air est constituée par un soufflet (10,110) qui peut être écrasé pratiquement totalement.

3. Appareil suivant la revendication 1 caractérisé en ce que la première section de tube est un coude.

4. Appareil suivant la revendication 1 caractérisé en ce que la partie mobile est constituée par un ballon (50) ou piston plongeur (56).

5. Appareil suivant l'une quelconque des revendications précédentes caractérisé en ce que le masque (20) est attaché à la première section de tube, à l'endroit de l'extrémité où se trouve son orifice de sortie d'air.

6. Appareil suivant l'une quelconque des revendications précédentes caractérisé en ce qu'il comporte un conduit rentrant (70) s'étendant dans la première section de tube (15) et définissant un évent de décharge d'air (71) destiné à communiquer avec l'orifice de sortie d'air (19) lorsque le second clapet (32) est fermé, ce second clapet comportant un volet (32a) et l'évent de décharge d'air (71) étant fermé par le volet (32a) du second clapet lorsque ce second clapet (32) est ouvert.

7. Appareil suivant la revendication 1 caractérisé en ce qu'il comporte une troisième section de tube (17), les deuxième (16) et troisième (17) sections de tube ayant chacune une forme de T et présentant chacune un orifice latéral et des orifices frontaux, l'orifice latéral de la seconde section (16) concordant avec l'un des orifices frontaux de la troisième section (17).

8. Appareil suivant la revendication 7 caractérisé en ce que le premier clapet (31) commande l'orifice latéral de la deuxième section (16) tandis que le second clapet (32) commande un orifice frontal de la seconde section se trouvant en concordance avec la première section.

9. Appareil suivant la revendication 7 caractérisé en ce qu'il comporte une source d'oxygène (90) communiquant avec la troisième section (17) par l'intermédiaire de son orifice latéral (91) ou de son autre orifice frontal (92).

10. Appareil suivant la revendication 1 caractérisé en ce qu'il comporte un conduit rentrant (70) s'étendant dans une section de tube (15) et définissant un évent de décharge d'air (71) communiquant avec l'orifice de sortie d'air (19) et un capuchon (80) commandant l'échappement de l'air à partir du conduit rentrant et monté mobile de manière à augmenter ou diminuer la dimension effective de l'évent de décharge de l'air.

11. Appareil suivant la revendication 2 caractérisé en ce que le soufflet (110) a une forme conique permettant, en cours d'utilisation et également pendant son stockage, son écrasement frontal, pratiquement complètement, réalisé manuellement.

**Patentansprüche**

1. Wiederbelebungsgerät, bestehend aus einem manuell zusammendrückbaren Luftgefäß (10) mit einem Auslaß (11) für eine Luftabgabe, einem ersten und einem zweiten Leitungsabschnitt (15,16), die in Reihe geschaltet sind und eine für einen Luftdurchgang mit dem Gefäßabgabeauslaß (11) verbindbare Lufteinlaßeinrichtung (18) besitzen, einem für einen Luftdurchgang mit einer auf das Gesicht eines Patienten aufzusetzenden Maske (20) verbindbaren Luftauslaß (19) und einem ersten und einem zweiten Klappenventil (31,32), die zumindest in einem Leitungsabschnitt (16) derart angeordnet sind, daß das erste Klappenventil (31) öffnet und Luft zu dem Gefäß (10) leitet, wenn das zweite Klappenventil (32) geschlossen ist, und das zweite Klappenventil (32) öffnet und Luft vom Gefäß zur Maske (20) leitet, wenn das Gefäß zusammengedrückt wird und das erste Klappenventil (31) geschlossen ist, gekennzeichnet durch eine vom ersten Abschnitt (15) gehaltene auf Luftdruck ansprechende Anzeige, um das Ausmaß des Druckaufbaus zwischen dem zweiten Ventil (32) und dem Luftauslaß (19) des Leitungsabschnitts anzuzeigen, wobei die Anzeige einen druckempfindlichen Teil (50;56), der beweglich vom ersten Abschnitt gehalten ist, und eine Einrichtung zum Anzeigen der Verlagerung des Teils infolge des Luftdruckaufbaus aufweist.

2. Wiederbelebungsgerät nach Anspruch 1, bei dem das Luftgefäß einen Balg (10,110) umfaßt, der im wesentlichen vollständig zusammendrückbar ist.

3. Wiederbelebungsgerät nach Anspruch 1, bei dem der eine Leitungsabschnitt ein Kniestück ist.

4. Wiederbelebungsgerät nach Anspruch 1, bei dem der Teil einen Ballon (50) oder einen Kolben (56) umfaßt.

5. Wiederbelebungsgerät nach einem der vorhergehenden Ansprüche, bei dem die Maske (20) am ersten Abschnitt an dessen Luftauslaßende angebracht ist.

6. Wiederbelebungsgerät nach einem der vorhergehenden Ansprüche, mit einem einspringenden Kanal (70), der sich in einen Leitungsabschnitt (15) hineinerstreckt und eine Luftaustrittsöffnung (71) bildet, die mit dem Luftauslaß (19) in Verbindung stehen soll, wenn das zweite Klappenventil (32) geschlossen ist, wobei das zweite Klappenventil eine Klappe (32a) aufweist und die Luftaustrittsöffnugn (71) von der Klappe (32a) des zweiten Klappenventils geschlossen ist, wenn das zweite Klappenventil (32) offen ist.

7. Wiederbelebungsgerät nach Anspruch 1, mit einem dritten Leitungsabschnitt (17), wobei der zweite (16) und der dritte Abschnitt (17) T-förmig sind und jeweils eine Seitenöffnung und endseitige Öffnungen besitzen und die Seitenöffnung des zweiten Abschnitts (16) mit einer endseitigen Öffnung des dritten Abschnitts (17) fluchtend übereinstimmt.

8. Wiederbelebungsgerät nach Anspruch 7, bei dem das erste Klappenventil (31) die Seitenöffnung des zweiten Abschnitts (16) steuert und das zweite Klappenventil (32) eine endseitige Öffnung des zweiten Abschnitts steuert, die mit dem ersten Abschnitt fluchtet.

9. Wiederbelebungsgerät nach Anspruch 7, mit einer Sauerstoffquelle (90) in Verbindung mit dem dritten Abschnitt (17) über dessen Seitenöffnung (91) oder dessen anderer endseitiger Öffnung (92).

10. Wiederbelebungsgerät nach Anspruch 1, mit einem einspringenden Luftaustrittskanal (70), der sich in einen Leitungsabschnitt (15) hineinerstreckt und eine Luftaustrittsöffnung (71) bildet, die mit dem Luftauslaß (19) in Verbindung steht, und mit einer Kappe (80), die das Entweichen von Luft aus dem einspringenden Kanal steuert und für eine Vergrößerung oder Verringerung der effektiven Größe der Luftaustrittsöffnung

11. Wiederbelebungsgerät nach Anspruch 2, bei dem der Balg (110) eine konische Form besitzt, was ein im wesentlichen vollständiges, vom Ende her manuell durchgeführtes Zusammendrücken im Gebrauch und auch für die Aufbewahrung ermöglicht.

FIG.1.

FIG.2.

FIG.4.

FIG.5.

EP 0 252 618 B1

FIG.3.

FIG.6.

FIG.7.

FIG.8.

FIG.9.

8